# EUROPEAN PATENT SPECIFICATION

(11) **EP 4 419 710 B1**
(45) Date of publication and mention of the grant of the patent: **02.07.2025**
(21) Application number: 22802478.2
(22) Date of filing: 20.10.2022
(51) Int. Cl.: C12Q 1/6832

(54) **PRE-LIBRARY TARGET ENRICHMENT FOR NUCLEIC ACID SEQUENCING**
VORBIBLIOTHEKSZIELANREICHERUNG ZUR NUKLEINSÄURESEQUENZIERUNG
ENRICHISSEMENT DES CIBLES DE PRÉ-BANQUE POUR LE SÉQUENÇAGE D'ACIDE NUCLÉIQUE

(30) Priority: 21.10.2021 US 202163270499 P
(43) Date of publication of application: 28.08.2024
(73) Proprietor: ILLUMINA, INC., San Diego, CA 92122 (US)
(72) Inventor: GALL, Alexander A., San Carlos, California 94070 (US); SCHLABERG, Robert, San Carlos, California 94070 (US)
(74) Representative: Williams, Andrea
(86) International application number: PCT/US2022/078452
(87) International publication number: WO 2023/070047

(56) References cited:
- WO-A1-2010/021702
- WO-A1-2017/049213
- WO-A1-2021/222289
- WO-A1-97/12896
- WO-A2-2007/127992
- US-A1- 2013 323 725
- KUTYAVIN I V: "Use of Base-Modified Duplex-Stabilizing Deoxynucleoside 5 '-Triphosphates To Enhance the Hybridization Properties of Primers and Probes in Detection Polymerase Chain Reaction", BIOCHEMISTRY, AMERICAN CHEMICAL SOCIETY, vol. 47, no. 51, 23 December 2008 (2008-12-23), pages 13666 - 13673, XP002569787, ISSN: 0006-2960, [retrieved on 20081201], DOI: 10.1021/BI8017784
- GUENTHER DALE C. ET AL: "Invader probes: harnessing the energy of intercalation to facilitate recognition of chromosomal DNA for diagnostic applications", CHEMICAL SCIENCE, vol. 6, no. 8, 1 January 2015 (2015-01-01), United Kingdom, pages 5006 - 5015, XP093008830, ISSN: 2041-6520, Retrieved from the Internet <URL:https://www.ncbi.nlm.nih.gov/pmc/articles/PMC4521421/pdf/SC-006-C5SC01238D.pdf> DOI: 10.1039/C5SC01238D
- I. V. SMOLINA: "End invasion of peptide nucleic acids (PNAs) with mixed-base composition into linear DNA duplexes", NUCLEIC ACIDS RESEARCH, vol. 33, no. 17, 25 September 2005 (2005-09-25), GB, pages e146 - e146, XP055336679, ISSN: 0305-1048, DOI: 10.1093/nar/gni151
- BUKANOV NIKOLAY O ET AL: "PD-loop: A complex of duplex DNA with an oligonucleotide (peptide nucleic acidaffinity capturehybridizationbiomagnetic separationyeast DNA)", BIOCHEMISTRY AND APPROVED MARCH, 1 May 1988 (1988-05-01), pages 5516 - 5520, XP093008931, Retrieved from the Internet <URL:https://www.ncbi.nlm.nih.gov/pmc/articles/PMC20409/pdf/pq005516.pdf> [retrieved on 20221216]
- RAJENDRAN ARIVAZHAGAN ET AL: "One-Pot Isolation of a Desired Human Genome Fragment by Using a Biotinylated pcPNA/S1 Nuclease Combination", BIOCHEMISTRY, vol. 57, no. 20, 22 May 2018 (2018-05-22), pages 2908 - 2912, XP093008988, ISSN: 0006-2960, DOI: 10.1021/acs.biochem.8b00202
- SUJAY P. SAU ET AL: "Invader LNA: Efficient targeting of short double stranded DNA", ORGANIC & BIOMOLECULAR CHEMISTRY, vol. 8, no. 9, 1 January 2010 (2010-01-01), pages 2028, XP055078396, ISSN: 1477-0520, DOI: 10.1039/b923465a
- ANDERSON BROOKE ET AL: "Mixed-Sequence Recognition of Double-Stranded DNA Using Enzymatically Stable Phosphorothioate Invader Probes", MOLECULES, vol. 20, no. 8, 1 January 2015 (2015-01-01), pages 13780 - 13793, XP093008899, Retrieved from the Internet <URL:https://www.ncbi.nlm.nih.gov/pmc/articles/PMC6332310/pdf/molecules-20-13780.pdf> DOI: 10.3390/molecules200813780
- GUENTHER DALE C. ET AL: "Bulged Invader probes: activated duplexes for mixed-sequence dsDNA recognition with improved thermodynamic and kinetic profiles", CHEMICAL COMMUNICATIONS, vol. 51, no. 81, 1 January 2015 (2015-01-01), UK, pages 15051 - 15054, XP093008900, ISSN: 1359-7345, Retrieved from the Internet <URL:https://www.ncbi.nlm.nih.gov/pmc/articles/PMC4589525/pdf/nihms719477.pdf> DOI: 10.1039/C5CC06264K
- ANDERSON BROOKE A. ET AL: "Merging Two Strategies for Mixed-Sequence Recognition of Double-Stranded DNA: Pseudocomplementary Invader Probes", THE JOURNAL OF ORGANIC CHEMISTRY, vol. 81, no. 8, 15 April 2016 (2016-04-15), pages 3335 - 3346, XP093008902, ISSN: 0022-3263, Retrieved from the Internet <URL:https://www.ncbi.nlm.nih.gov/pmc/articles/PMC4836393/pdf/jo6b00369.pdf> DOI: 10.1021/acs.joc.6b00369
- CHANDLER D P ET AL: "AFFINITY PURIFICATION OF DNA AND RNA FROM ENVIRONMENTAL SAMPLES WITH PEPTIDE NUCLEIC ACID CLAMPS", APPLIED AND ENVIRONMENTAL MICROBIOLOGY, AMERICAN SOCIETY FOR MICROBIOLOGY, US, vol. 66, no. 8, 1 August 2000 (2000-08-01), pages 3438 - 3445, XP001162781, ISSN: 0099-2240, DOI: 10.1128/AEM.66.8.3438-3445.2000
- KUTYAVIN I V ET AL: "Oligonucleotides containing 2-aminoadenine and 2-thiothymine act as selectively binding complementary agents", BIOCHEMISTRY,, vol. 35, no. 34, 1 January 1996 (1996-01-01), pages 11170 - 11176, XP002217382, ISSN: 0006-2960, DOI: 10.1021/BI960626V

## Description

### CROSS-REFERENCE TO RELATED APPLICATIONS

This application claims priority to U.S. Provisional Application No. 63/270,499, filed October 21, 2021.

### SEQUENCE LISTING

The present application is being filed along with a Sequence Listing in electronic format. The Sequence Listing is provided as a file entitled ILLINC752.xml, created and last saved on October 13, 2022, which is 79,672 bytes in size..

### TECHNICAL FIELD

Novel selectively binding complementary (SBC) oligodeoxynucleotides (ODNs) and their use in enriching targeted dsDNA for sequencing-based detection and genomic profiling.

### BACKGROUND

Detecting specific microorganisms and characterizing their genetic makeup in complex biological samples is of great importance in human and veterinary medicine, agriculture, and other fields. DNA sequencing-based approaches - particularly next-generation sequencing - vastly improve breadth of detection and resolution of genotypic characterization over conventional culture-based or nucleic acid amplification-based methods.

Methods for efficient sequencing-based detection and genomic profiling of microorganisms from complex biological specimens often rely on enrichment of the relevant microbial DNA. Without this step, both targeted DNA of interest and non-relevant background DNA are sequenced non-selectively. Enrichment can be performed by single or multiplex PCR (amplicon sequencing) or DNA probe-based capture of relevant microbial DNA targets (target capture). For amplicon sequencing, targeted DNA sequences are enriched by PCR amplification directly from the complex mixture of microbial and background DNA. For target capture, the complex admixture of microbial and background DNA is first converted into a sequencing library in a non-selective way (shotgun library). Targeted microbial DNA is then captured from a defined (small) amount of the shotgun library with fully or partially complementary DNA probes to enrich and then subject captured DNA to next-generation sequencing. Enriching targeted DNA prior to sequencing increases the ratio of relevant (targeted) to non-relevant (background) DNA and improves efficiency.

Target capture is an effective method when the relevant DNA is present in the complex admixture at sufficient abundance to be captured from the defined, small amount of the shotgun library used in the capture reaction. However, in samples with very high concentrations of background DNA (e.g., in DNA extracted from tissue samples), relevant microbial DNA may be drowned out and not present or present at insufficient concentrations in the defined, small amount of sequencing library for efficient capture (i.e., the capture reaction may only contain background DNA due to its high abundance). In these cases, performing the target capture step prior to preparing the sequencing library, from a much larger fraction of the sample DNA, ensures that the relevant microbial DNA is enriched relative to the background DNA and then available for downstream sequencing.

WO2017049213A1 describes compositions and methods for the enrichment of a multiplicity of long DNA sequences selected from the genome of any eukaryote. Capture is performed using multiple PNA molecules with gamma-modified chiral backbones, comprising a mixture of neutral and positive chemical groups such as diethylene glycol, gamma-L-lysine, gamma-L-thialysine. Two or more PNA probes with covalently bound haptens, preferably biotin, target each DNA domain of interest for capture, isolation, and subsequent sequencing analysis of the multiplicity of enriched targets, including DNA methylation sequencing. The methods include enhancement of probe-DNA binding specificity through single strand binding proteins (SSB).

US2013323725A1 describes a method comprising: a) clamping the top and bottom strands of a double stranded DNA molecule to produce a duplex in which the top and bottom strands are linked; b) denaturing the duplex to produce a denatured product; and c) renaturing the denatured product in the presence of a labeled oligonucleotide that is complementary to a sequence of nucleotides in the double stranded DNA molecule, thereby producing a D-loop-containing product.

Despite the advances in sequencing-based detection and genomic profiling of microorganisms from complex biological specimens, a need exists for improved reagents and methods for enrichment of the relevant microbial DNA coupled with sequencing-based detection and genomic profiling.

### SUMMARY

The present disclosure describes selectively binding complementary (SBC) oligodeoxynucleotide (ODN) derivatives and their use in enriching targeted dsDNA for sequencing-based detection and genomic profiling.

In one aspect, the invention provides selectively binding complementary oligodeoxynucleotide derivatives ("SBC probes"). In certain embodiments, the selectively binding complementary oligodeoxynucleotide derivatives have the structure: 5'-(Affinity tag)-(SBC sequence 2) - (Spacer) - (SBC sequence 1)-(Blocking group or OH)-3' wherein the 3'-blocking group is no blocking (3'-OH), or any alkene diol (e.g., OCH₂CH₂CH₂OH), or another biotin with a linker, or stabilizing group (MGB, or intercalators such as phenazinium, acridine, pyrene); SBC sequences 1 and 2, each is 5 - 100 bases long with strands complementary to a selected region of larger targeted dsDNA, such as the targeted DNA preserves a partial double stranded structure after invasion of SBC probe; and 5'-(Affinity tag) is an affinity tag connected to 5'-end of the probe by a linker. As used herein, the term "affinity tag" refers a moiety this is recognized and bound by its affinity partner thereby allowing the SBC probe bearing the tag to be isolated via the binding interaction between the affinity tag and its affinity partner. Representative affinity tags useful in the SBC probes described herein are known in the art. In certain embodiments, the affinity tag is biotin connected to 5'-end of the probe by a linker. For biotin and biotin-like affinity tags, the affinity binding partner is an avidin (e.g., streptavidin).

Suitable linkers useful in the SBC probes include nucleotide sequences having approximately 1 to 10 nucleotides of DNA or RNA sequence, such as oligo dT, oligo dA or other selections that can avoid undesired hydrogen bonding. A specific example for the tether would be an ODN moiety having four T's. Alternatively, the tethering linkage may comprise the grouping -[OCH₂-CH₂]ₙ-O-, where n is 1 to 10 or one to three inserts of -(CH₂)ₙ-linkers separated by phosphate groups, where n is 2 to 10.

In certain embodiments, the linkers include stabilizing moieties, such as intercalators (phenazinium, acridine, pyrene).

In certain embodiments, the SBC sequences comprise 2-sT - 2-amA base pairs.

In other embodiments, the SBC sequences comprise W - 2-amA base pairs.

In further embodiments, the SBC sequences comprise both 2-sT - 2-amA and W - 2-amA base pairs.

In another aspect, the invention provides methods for enriching targeted dsDNA in a DNA sample. In certain embodiments, the method comprises:
(a) mixing a selectively binding complementary (SBC) probe with dsDNA extracted from sample or cDNA produced from corresponding RNA, targeted and non-targeted, wherein the SBC probe is composed of two complementary weakly bound strands tethered with a linker and containing an affinity tag (e.g., biotin label);
(b) hybridizing each strand of SBC probe with the targeted region of dsDNA and forming two strong double stand areas (double D-loop) within the targeted dsDNA or cDNA;
(c) treating the mixture with affinity partner-labeled (e.g., streptavidin-labeled) beads (or other solid phase or substrate) and binding the hybridized biotinylated probe to the beads;
(d) separating the beads containing targeted DNA bound to the beads from non-targeted DNA in the solution and washing the beads; and
(e) melting off the probe while protecting the rest of the targeted DNA in a double stranded form to provide an enriched dsDNA pool.

In certain embodiments, the method further comprising subjecting the enriched dsDNA pool to standard library prep for the sequencing.

In certain embodiments, the SBC probe is an oligodeoxynucleotide derivative as described herein.

In certain embodiments of the method, recA enzyme is used strand exchange of SBC probe with targeted dsDNA for capturing and enrichment.

The invention provides the use of a selectively binding complementary oligodeoxynucleotide derivative as described herein for enriching targeted dsDNA in a sample for sequencing-based detection and genomic profiling of the targeted dsDNA.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings.
Fig. 1 illustrates a representative biotin labeled SBC probe forming stable duplex with one strand of a double stranded nucleic acid target leading to formation of D-loop.
Fig. 2 illustrates two complementary biotin labeled SBC probes with a double stranded nucleic acid target leading to formation of two stable D-loops.
Fig. 3 illustrates two complementary biotin-labeled and tethered with flexible linker SBC probes forming two stable complementary hybrids with a double stranded nucleic acid target leading to formation of two stable D-loops.
Fig. 4 illustrates steps of a representative enrichment method disclosed herein, with biotin as the affinity tag and streptavidin as the affinity partner:
   (a) mixing SBC probe with dsDNA extracted from sample or cDNA produced from corresponding RNA, targeted and non-targeted. SBC probe is composed of two complementary weakly bound strands tethered with a linker and containing a biotin label, as the affinity tag;
   (b) hybridizing each strand of SBC probe with the targeted region of dsDNA and forming two strong double stand areas (double D-loop) within the targeted dsDNA or cDNA;
   (c) treating the mixture with streptavidin (affinity partner) beads and binding the hybridized biotinylated probe to the beads;
   (d) separating the beads containing targeted DNA bound to the beads from non-targeted DNA in the solution and wash the beads;
   (e) melting off the probe while protecting the rest of the targeted DNA in a double stranded form; and
   (f) subjecting the enriched dsDNA pool to standard library prep for the sequencing.

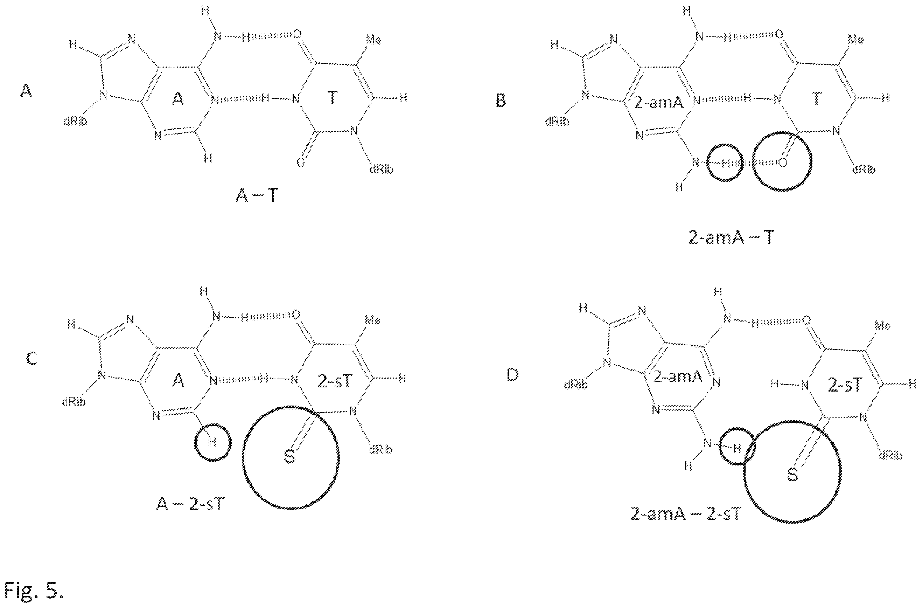

Figs. 5A-5D are illustrations of hydrogen bonds formation and disruption between natural and modified nucleotides: A - T base pair that forms two hydrogen bonds (5A); 2-amA - T base pair that forms three hydrogen bonds (5B); A - 2-sT base pair that forms two hydrogen bonds and additionally stabilized by van der Waals attraction of large sulfur atom with 2-H of adenine base (5C); 2-amA - 2-sT bases are sterically hindered because of van der Waals repulsion between a large sulfur atom of 2-thiothymidine and 2-amino group of adenine base (5D). The distance between N-H and N atoms becomes too large for formation of the second hydrogen bond.

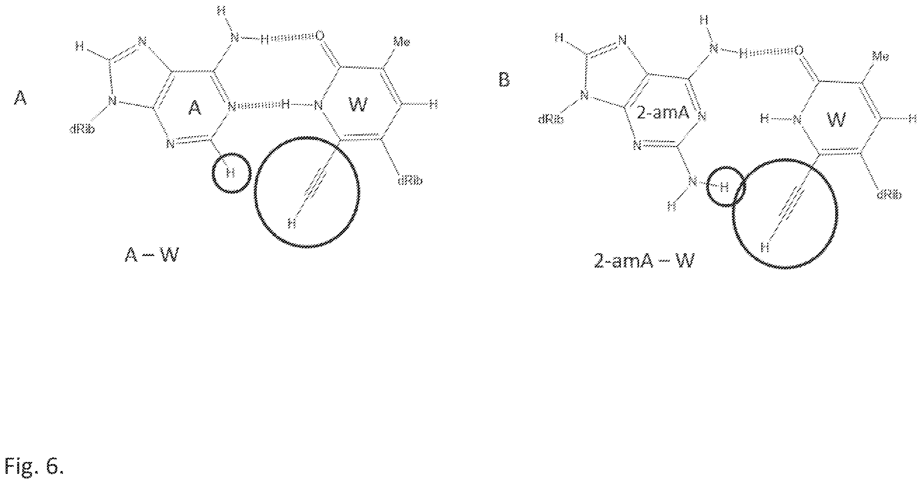

Figs. 6A and 6B illustrate A - W base pairs. A - W base pair that forms two hydrogen bonds and additionally stabilized by van der Waals attraction of 2-akyne substituent with 2-H of adenine base (6A); 2-amA - W base that is sterically disrupted in formation of two hydrogen bonds by a bulky alkyne substituent of W interacting with 2-amino group of adenine base (6B).

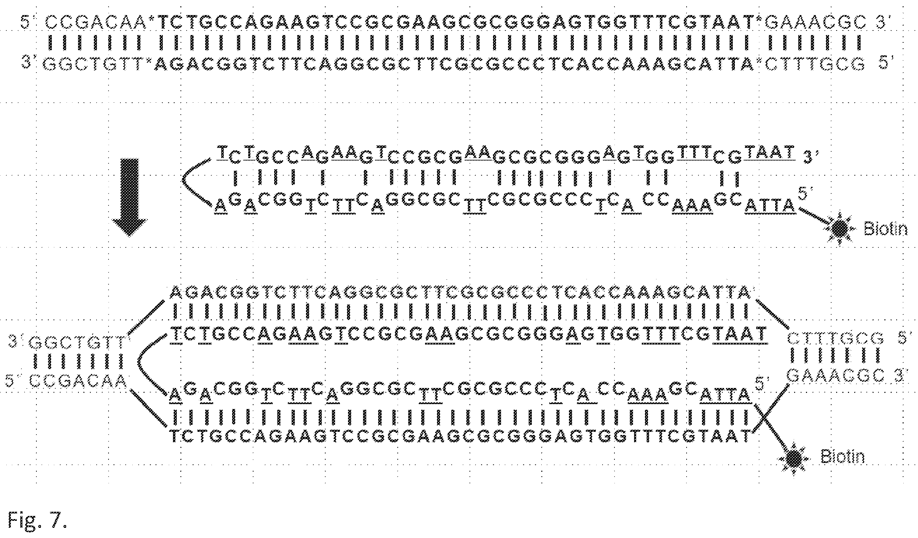

Fig. 7 illustrates a representative process of hybridization of SBC probe targeting double strand targeted region of Seq. 1 and corresponding region of the complementary strand. SBC probe is composed of two complementary sequences with T replaced with 2-sT or W and A replaced with 2-amA. All modified bases are underlined. Both SBC sequences are connected through a flexible linker (Spacer) and the whole probe is terminated with biotin label at the 5-end.

### DETAILED DESCRIPTION

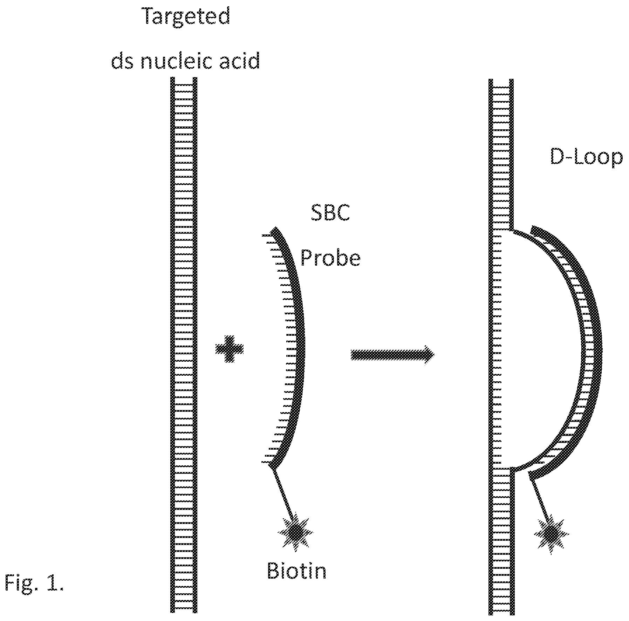

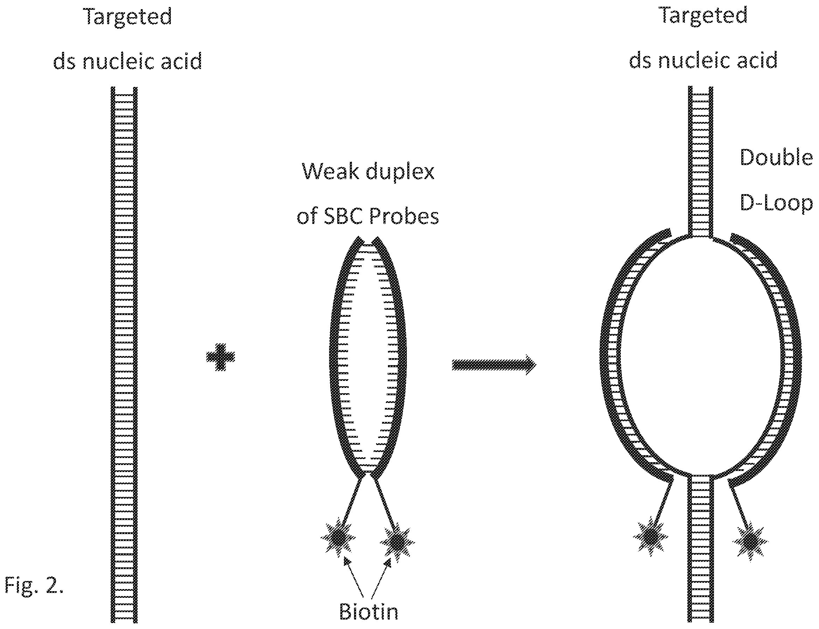

The present disclosure describes a method for selective capturing of targeted dsDNA without complete dissociation of the targeted strands. In the method, we utilize a partial disruption of the double strand structure of targeted dsDNA by formation of more thermodynamically stable complementary D-loop (Fig. 1) or double D-loop (Fig. 2). Such probes are specially designed to bind stronger to the complementary natural targets than to themselves. Such property can be provided by modified base pairs as described in U.S. Patent No. 5,912,340, entitled "Selective binding complementary oligonucleotides. The method utilizes unique binding properties of Selective Binding Complementary (SBC) oligonucleotides that form stronger complementary complexes with natural DNA than binding to itself. U.S. Patent No. 5,912,340, discloses that strand invasion and double D-loop formation in long DNA by paired SBC ODNs can be catalyzed by recombinase enzymes such as recA.

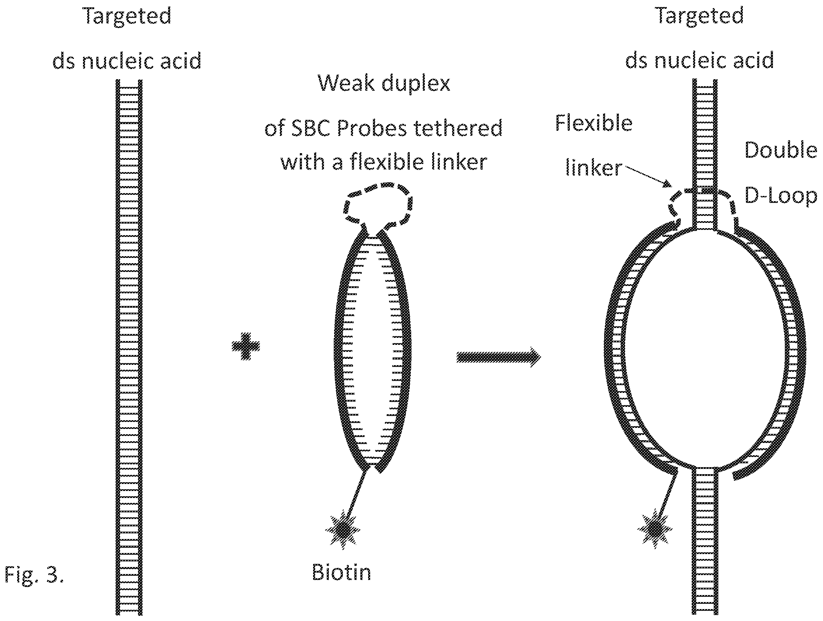

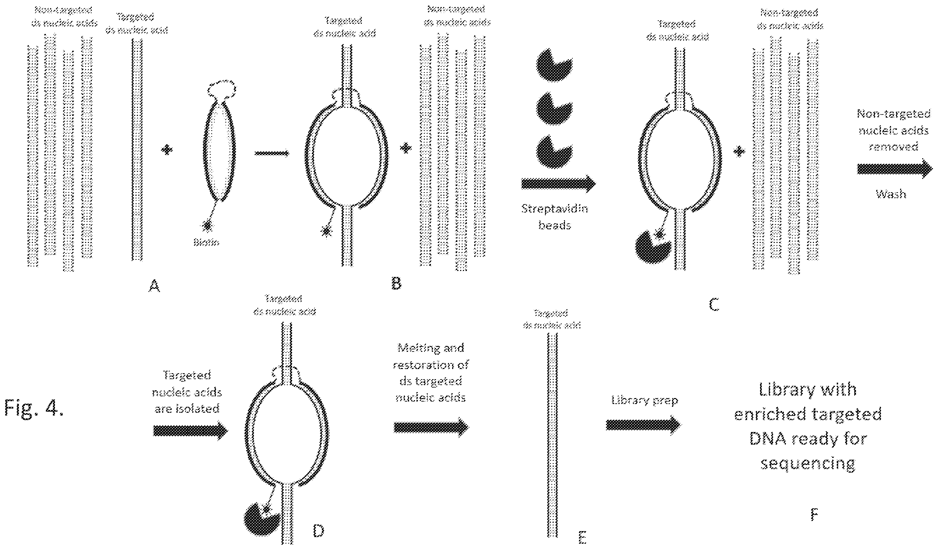

As described herein, sequence specific invasion of targeted dsDNA is utilized for enrichment of the targeted dsDNA by partial disruption of its structure by SBC probes containing biotin label. In one embodiment, complementary strands of probes which are both weakly bound to themselves are linked by a flexible linker allowing simultaneous hybridization with both strands of the targeted region of the dsDNA. Fig. 3 depicts invasion of the double strand structure of targeted DNA by formation of two more stable duplexes in a double D-loop structure. In all cases, the probes are labelled with biotin for selective extraction of the targeted dsDNA from the mixture with non-targeted DNA. In clinical samples, the majority of non-targeted DNA is represented by the host DNA. All host DNA that is still present after selective extraction of the targeted DNA will be subjected to the library prep and eventually sequenced along with the targeted DNA. Maximizing the rate of enrichment of the targeted DNA vs. untargeted DNA is important for improvements of sensitivity and for faster data analysis. Fig. 4 depicts the following steps of enrichment:
(a) mixing SBC probe with dsDNA extracted from sample or cDNA produced from corresponding RNA, targeted and non-targeted. The SBC probe is composed of two complementary weakly bound strands tethered with a linker and containing an affinity tag (e.g., biotin label);
(b) hybridizing each strand of SBC probe with the targeted region of dsDNA and forming two strong double stand areas (double D-loop) within the targeted dsDNA or cDNA;
(c) treating the mixture with affinity partner-labeled (e.g., streptavidin-labeled) beads (or other solid phase or substrate) and binding the hybridized biotinylated probe to the beads;
(d) separating the beads containing targeted DNA bound to the beads from non-targeted DNA in the solution and washing the beads;
(e) melting off the probe while protecting the rest of the targeted DNA in a double stranded form; and
(f) subjecting the enriched dsDNA pool to standard library prep for the sequencing.

Selective binding of 2-thio-dT (2-sT) and 2-amino-dA (2-amA) to corresponding natural bases A and T bases and not to themselves is possible due to the steric hindrance created by a large sulfur atom replacing oxygen atom in the 2-position of thymidine. While natural A-T base pair (Fig. 5A) has two hydrogen bonds, replacing hydrogen atom in the 2-position of adenine with amino group enables even stronger binding by creating three hydrogen bonds between 2-amA and T (Fig. 5B). Additional hydrogen bonds lead to a stronger binding of 2-amA-modified probe to the natural complementary DNA. As shown by I.V. Kutyavin, et al., Biochemistry, 1996, 35, 11170-11176, incorporation of five 2-amA-bases in place of natural dA in a 20-mer probe increases its binding to the complementary oligo target by 4°. When five natural dT bases in 20-mer probe were replaced by 2-sT, mild stabilization by 2° is observed. Such slight stabilization by a larger sulfur atom in the 2-position of dT is explained by Herman O. Sintim and Eric T. Kool, J Am Chem Soc. 2006 Jan 18; 128(2): 396-397,. The authors found that the larger size can lead to increased efficiency and selectivity in pairing and replication (Fig. 5C). Design of SBC probes as shown on Fig. 2 and Fig. 3 requires positioning of both modified bases 2-sT and 2-amA across each other in complementary sequences. SBC probes are composed of complementary DNA strands with all or partial or single replacement of dT - A base pairs to corresponding 2-sT - 2-amA base pairs. When partial replacement is desired, all modifications should be positioned across each other in the complementary sequences of SBC probes. As disclosed by above cited I.V. Kutyavin, *et al.,* model building indicates that steric clash between the 2-thio group of thymine and the 2-amino group of adenine tilts the bases relative to each other, thereby allowing only one hydrogen bond to form, as shown on Fig, 5D. In experiments where the authors replaced all five dT and all five dA in both strands of the modified oligonucleotides, the melting temperature of such complementary 20-mer oligos fell by 29° (from 55° to 26°). At the same time, binding of each strand to complementary oligonucleotides composed of all natural bases demonstrated increasing melting temperature by 8-10° (to 63° and 65°). Such dramatic difference between self-binding and binding to natural targets provides a tool that is capable of invading double stranded DNA target by formation of one (Fig. 1) or two (Fig.2) more stable D-loops in a sequence-specific manner. In an alternative embodiment, both strands of the complementary SBC sequences are linked (tethered) head-to-tail on corresponding 5'- and 3'-ends by a flexible linker (Fig. 3). The covalent linkage does not participate in hybridization and does not prevent hybridization of the two SBC members to the two strands of the targeted sequence. In such designs, both strands are becoming available for binding in the same proximity to both strands of the targeted dsDNA. The latter design is preferred, as it will give maximal thermodynamic and kinetic benefits to the enrichment process. The linker is preferentially composed of polyethylene glycol chains, but it can be made of any linking groups which are suitable as "tethers" for linking the two SBC ODNs of a matched pair to one another. Such linkers include nucleotide sequences having approximately 1 to 10 nucleotides of DNA or RNA sequence, such as oligo dT, oligo dA or other selections that can avoid undesired hydrogen bonding. A specific example for the tether would be an ODN moiety having four T's. Alternatively, the tethering linkage may comprise the grouping -[OCH₂-CH₂]ₙ-O-, where n is 1 to 10 or one to three inserts of -(CH₂)ₙ- linkers separated by phosphate groups, where n is 2 to 10.

In some embodiments, linkers contain stabilizing moieties such as intercalators (phenazinium, acridine, pyrene). One or both ends of the SBC probes are labeled with biotin for selective capturing of the hybridized probe after invasion of the targeted dsDNA. Steps of the enrichment method are shown of Fig. 4: hybridization to the targeted dsDNA in the presence of untargeted DNA (A and B), capture with streptavidin magnetic beads (C), separation of untargeted DNA by wash (D), release of the targeted DNA by melting off the SBC probe and restoration of a double strand structure (E) with a subsequent sequencing library prep (F).

Alternatively, SBC probes are composed of complementary DNA strands with all or partial or single replacement of dT - A base pairs to corresponding W - 2-amA base pairs. When partial replacement is desired, all modifications should be positioned across each other in the complementary sequences of SBC probes. W modification can be introduced using a corresponding dW phosphoramidite reagent commercially available from Glen Research:

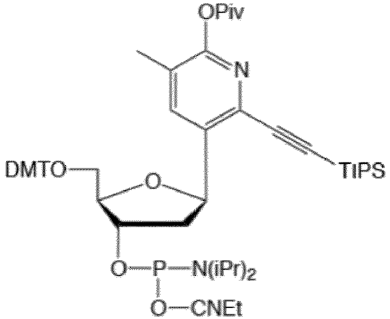

### dW-CE Phosphoramidite (Glen Research cat. no 10-1527)

The phosphoramidite allows for introduction of W into oligonucleotides by automated DNA synthesis using conventional chain extension cycles. The pivaloyl (Piv) group is easily removed with the ammonia solution used to deprotect the natural nucleobases. Removal of the triisopropylsilyl (TIPS) protecting group within the ethynyl group is induced under conditions similar to those used for 2'-TBDMS protected RNA strands, with tetrabutylammonium fluoride (TBAF) as deprotection agent.

A W modified base was initially reported by T.J. Walter, and C. Richert, in A strongly pairing fifth base: oligonucleotides with a C-nucleoside replacing thymidine Nucleic Acids Res., 2018, 46, 8069-8078,. The authors called it a W-base. They have demonstrated that a single replacement of thymidine base in a 12-mer complementary duplex DNA increases melting temperature by 4.4°. The Stabilizing effect is essentially additive and not sensitive to positioning of W-modification in a row or apart. In one instance, replacement of six T bases to W in the 11-mer increased binding by 17.5°. Such stabilizing effect, according to the authors, brings stability of A - W pair to the level of G - C pair, and they speculated that the ethynyl group at position 2 is well suited to interact with the lower Watson-Crick face of adenine, with a likely van der Waals contact to the CH fragment at position 2 of the purine, as shown in Fig. 6A.

While A - W base pair is stabilized by van der Waals interaction, we found that alkyne group creates steric hindrance, when W base is positioned against 2-amA base as shown on Fig. 6B. That substantial discrepancy of W base between stabilizing pairing with natural A and destabilizing binding with modified base 2-amA creates an alternative way of designing affective SBCs analogous to utility of 2-sT and 2-amA pair.

Representative SBC probes described herein include three designs: (1) containing only 2-sT - 2-amA base pairs, (2) containing only W - 2-amA base pairs, and (3) mixed designs containing both 2-sT - 2-amA and W - 2-amA base pairs.

The following examples are provided for the purpose of illustrating, not limiting, the invention.

### EXAMPLES

### Materials and methods

Oligonucleotide probes are synthesized using ordinary phosphoramidite chemistry on oligonucleotide synthesizer. Shasta synthesizer (Sierra BioSystems, Inc., Sonora, CA) is used for making all oligonucleotides. SBC probes with modified bases are synthesized using the following commercial reagents from Glen Research: dW phosphoramidite (cat. no 10-1527); 2-Thio-dT-CE Phosphoramidite (cat. no. 10-1036-02); 2-Amino-dA-CE Phosphoramidite (cat. no 10-1085-02); Spacer Phosphoramidite C3 (cat. no. 10-1913-02); Spacer Phosphoramidite 9 (cat. no. 10-1909-02); Spacer Phosphoramidite 18 (cat. no. 10-1918-02); 5'-Biotin Phosphoramidite (cat. no. 10-5950-02). Syntheses and deprotections of modified oligonucleotides are performed by following Glen Research's recommendations for all modified nucleosides.

SBC probes for the experiments are designed as 2x40-mers separated by a spacer with sequences complementary to both strands of expected targets. In one example, the synthesis is performed at a 50 nM scale using columns packed with 2000A Uni support from Biocomma Ltd. (China), cat. No. DS0050-2-3900. This method produces probes with free 3'-OH group. Another set of probes is synthesized on a 3'-Spacer C3 CPG1000 from AM Chemicals LLC (4065 Oceanside Blvd., Suite M Oceanside, CA 92056-5824) in a 0.2 mM scale. 3'-blocking C3 group maybe useful for prevention of accidental extension in the subsequent PCR steps. SBC sequences in the probes were separated by various linkers using Spacer Phosphoramidite, and the syntheses in all examples are terminated by introduction of biotin label at the 5'-end using 5'-Biotin Phosphoramidite. Modifications after incorporation into oligos of the reagents are coded as follows:

| | |
|---|---|
| dW phosphoramidite | W |
| 2-Thio-dT-CE Phosphoramidite | 2-sT |
| 2-Amino-dA-CE Phosphoramidite | 2-amA |
| Spacer Phosphoramidite C3 | S3 |
| Spacer Phosphoramidite 9 | S9 |
| Spacer Phosphoramidite 18 | S18 |
| 5'-Biotin Phosphoramidite | Biotin |
| 3'-Spacer C3 CPG1000 | C3 |

### General structure of tested SBC probes

Representative SBC probes have the following general structure: 5'-(Biotin)-(SBC sequence 2) - (Spacer) - (SBC sequence 1)-(Blocking group or OH)-3'

Both SBC sequences 1 and 2 are complementary to corresponding regions of targeted dsDNA. SBC sequences are synthesized with pairs of 2-sT and 2-amA modifications and with pairs of W and 2-amA modification. Another example demonstrates design with all three modified bases in the same SBC probe: 2-sT and W in place of T and 2-amA in place of A.

### Demonstration of selective binding to three E. coli targets

Three E. coli random coding regions of sizes between 500-1000 bp (582, 819, and 915 bp) Seq.1, Seq. 2 and Seq. 3 are selected for the demonstration (only one strand shown in each sequence). The targeted regions are flanked with asterisk and highlighted bold. Three 40 bp regions are selected for targeting. All sequences are shown from 5' to 3'-end.

Fig. 7 depicts the reaction of hybridization of SBC probe targeting double strand target of Seq. 1 and corresponding region of the complementary strand.
***Seq. 1.*** 
ATGAATTTTTTAGCTCACCTGCATTTAGCCCATCTCGCGGAAAGCTCGCTTTCCGGCAATTT
ACTGGCTGATTTCGTACGCGGAAATCCTGAAGAAAGTTTTCCGCCCGACGTCGTGGCTGGCA
TTCATATGCATCGGCGAATCGACGTATTAACCGACAA*TCTGCCAGAAGTCCGCGAAGCGCG
GGAGTGGTTTCGTAAT*GAAACGCGCCGTGTCGCGCCTATTACGCTGGATGTGATGTGGGAT
CACTTTCTGTCCCGCCACTGGTCGCAGCTGTCGCCCGATTTTCCGCTACAGGAATTTACCTG
TTATGCCCGGGAGCAAGTGATGACTATTCTTCCAGACTCACCGCCACGCTTTATCAATCTGA
ACAACTACTTATGGTCAGAGCAGTGGTTGGTGCGCTATCGCGATATGGATTTCATCCAGAGC
GTGTTAAACGGCATGGCAAGCCGTCGCCCACGTCTGGATGCCCTGCGTGACTCCTGGTACGA
TTTAGACGCCCATTACGATGCTCTCGAAACCCGCTTCTGGCAGTTTTATCCGCGGATGATGG
AGCAGGCGTCACGCAAGGCGTTATAA (SEQ ID NO:1)
***Seq. 2.*** 
ATGCAGCTAAGAATTACCAGTCGAAAAAAACTCACCGCTCTATTATGCGCTCTTGGGCTAAT
TTCCATCGTTGCAATTTATCCGCGTCAGACGGTGAATTTTTTCTATTCGACAGCAGTTCAGA
TTACAGACTACATCCACTTCTACGGTTATCGCCCGGTTAAATCTTTCGCTATTCATATTCCT
GCCAGTTACACCATTCACGGAATAGATGTTTCGCGCTGGCAGGAGCGGATCGACTGGCAGCG
GGTGGCAAAAATGCGCGATAACGGTATCCGCTTACAATTTGCCTTTATCAAGGCGACGGAAG
GCGAAAAGCTGGTGGACCCCTATTTTTCGCGTAACTGGCAACTAAGCCGCGAAAATGGCCTG
CTGCGCGGGGCGTATCATTATTTCTCCCCGT*CGGTAGCAGCGCCTGTTCAGGCGCGATTAT
TTCTGCAAAC*GGTGGATTTCTCACAAGGCGATTTCCCTGCCGTGCTGGACGTAGAAGAACG
GGGAAAATTATCGGCAAAAGAATTACGCAAGCGGGTAAGTCAGTGGCTAAAAATGGTCGAAA
AAAGTACGGGAAAAAAGCCGGTTATTTACTCAGGAGCCGTTTTTTATCACACCAATCTGGCG
GGCTATTTCAATGAATATCCGTGGTGGGTGGCTCACTACTATCAACGTCGCCCGGACAATGA
CGGCATGGCCTGGCGTTTCTGGCAGCATTCCGACCGTGGGCAGGTAGATGGCATTAATGGTT
CGGTGGATTTTAATGTGTTTAATGGCACAGAGGAGGAGTTGCAGGCATTCGTTGATGGGATT
AAAGAAACGCCTTAA (SEQ ID NO:2)
***Seq. 3.***

ATGAGCTGGATTGAACGAATTAAAAGCAACATTACTCCCACCCGCAAGGCGAGCATTCCTGA
AGGGGTGTGGACTAAGTGTGATAGCTGCGGTCAGGTTTTATACCGCGCTGAGCTGGAACGTA
ATCTTGAGGTCTGTCCGAAGTGTGACCATCACATGCGTATGACAGCGCGTAATCGCCTGCAT

AGCCTGTTAGATGAAGGAAGCCTTGTGGAGCTGGGTAGCGAGCTTGAGCCGAAAGATGTGCT
GAAGTTTCGTGACTCCAAGAAGTATAAAGACCGTCTGGCATCTGCGCAGAAAGAAACCGGCG
AAAAAGATGCGCTGGTGGTGATGAAAGGCACTCTGTATGGAATGCCGGTTGTCGCTGCGGCA
TTCGAGTTCGCCTTTATGGGCGGTTCCATGGGGTCTGTGGTAGGTGCGCGTTTCGTGCGTGC
CGTTGAGCAGGCGCTGGAAGATAACTGCCCACT*GATCTGCTTCTCCGCCTCTGGTGGCGCA
CGTATGCAGGAA*GCACTGATGTCGCTGATGCAGATGGCGAAAACCTCTGCGGCACTGGCAA
AAATGCAGGAGCGCGGCTTGCCGTACATCTCCGTGCTGACCGACCCGACCATGGGCGGTGTT
TCTGCAAGTTTCGCCATGCTGGGCGATCTCAACATCGCTGAACCGAAAGCGTTAATTGGCTT
TGCCGGTCCGCGTGTTATCGAACAGACTGTTCGCGAAAAACTGCCGCCAGGATTCCAGCGCA
GTGAATTCCTGATCGAGAAAGGCGCGATCGACATGATCGTCCGTCGTCCGGAAATGCGTCTG
AAACTGGCGAGCATTCTGGCGAAGTTGATGAATCTGCCAGCGCCGAATCCTGAAGCGCCGCG
TGAAGGCGTAGTGGTGCCACCGGTACCGGATCAGGAACCTGAGGCCTGA (SEQ ID NO:3)

The following are designs of SBC probes to all three Seq. 1-3. The designs vary by a type of Thymidine replacement (2-sT or W), by a blocking (or no blocking) at the 3'-end and by a length of a spacer. All tested designs are shown in Table 1. Modified bases underlined: T is 2-sT and A is 2-amA. Table 2. Modified bases underlined: T is 2-sT, W is replacing T or some of the T bases with W-base, and A is 2-amA.

**TABLE 1**

| **STRUCTURE OF PROBES 5'-(BIOTIN)-(SBC SEQUENCE 2) - (SPACER) - (SBC SEQUENCE 1) - (BLOCKING GROUP OR OH)-3'** | | | | |
|---|---|---|---|---|
| **SBC** | **SBC sequence 2** | **Spacer** | **SBC sequence 1** | **3'-** |
| 1a | ATTACGAAACCACTCCCGCGCTTCGCGGACTTCTGGCAGA | S3 | TCTGCCAGAAGTCCGCGAAGCGCGGGAGTGGTTTCGTAAT | OH |
| 1b | GTTTGCAGAAATAATCGCGCCTGAACAGGCGCTGCTACCG | S3 | CGGTAGCAGCGCCTGTTCAGGCGCGATTATTTCTGCAAAC | OH |
| 1c | TTCCTGCATACGTGCGCCACCAGAGGCGGAGAAGCAGATC | S3 | GATCTGCTTCTCCGCCTCTGGTGGCGCACGTATGCAGGAA | OH |
| 2a | ATTACGAAACCACTCCCGCGCTTCGCGGACTTCTGGCAGA | S3 | TCTGCCAGAAGTCCGCGAAGCGCGGGAGTGGTTTCGTAAT | C3 |
| 2b | GTTTGCAGAAATAATCGCGCCTGAACAGGCGCTGCTACCG | S3 | CGGTAGCAGCGCCTGTTCAGGCGCGATTATTTCTGCAAAC | C3 |
| 2c | TTCCTGCATACGTGCGCCACCAGAGGCGGAGAAGCAGATC | S3 | GATCTGCTTCTCCGCCTCTGGTGGCGCACGTATGCAGGAA | C3 |
| 3a | ATTACGAAACCACTCCCGCGCTTCGCGGACTTCTGGCAGA | S9 | TCTGCCAGAAGTCCGCGAAGCGCGGGAGTGGTTTCGTAAT | C3 |
| 3b | GTTTGCAGAAATAATCGCGCCTGAACAGGCGCTGCTACCG | S9 | CGGTAGCAGCGCCTGTTCAGGCGCGATTATTTCTGCAAAC | C3 |
| 3c | TTCCTGCATACGTGCGCCACCAGAGGCGGAGAAGCAGATC | S9 | GATCTGCTTCTCCGCCTCTGGTGGCGCACGTATGCAGGAA | C3 |
| 4a | ATTACGAAACCACTCCCGCGCTTCGCGGACTTCTGGCAGA | S18 | TCTGCCAGAAGTCCGCGAAGCGCGGGAGTGGTTTCGTAAT | C3 |

| **SBC** | **SBC sequence 2** | **Spacer** | **SBC sequence 1** | **3'-** |
|---|---|---|---|---|
| 4b | GTTTGCAGAAATAATCGCGCCTGAACAGGCGCTGCTACCG | S18 | CGGTAGCAGCGCCTGTTCAGGCGCGATTATTTCTGCAAAC | C3 |
| 4c | TTCCTGCATACGTGCGCCACCAGAGGCGGAGAAGCAGATC | S18 | GATCTGCTTCTCCGCCTCTGGTGGCGCACGTATGCAGGAA | C3 |

**TABLE 2**

| **STRUCTURE OF PROBES 5'-(BIOTIN)-(SBC SEQUENCE 2) - (SPACER) - (SBC SEQUENCE 1)-(BLOCKING GROUP OR OH)-3'** | | | | |
|---|---|---|---|---|
| **SBC** | **SBC sequence 2** | **Spacer** | **SBC sequence 1** | **3'-** |
| 5a | AWWACGAAACCACWCCCGCGCWWCGCGGACWWCWGGCAGA | S3 | WCWGCCAGAAGWCCGCGAAGCGCGGGAGWGGWWWCGWAAW | OH |
| 5b | GWWWGCAGAAAWAAWCGCGCCWGAACAGGCGCWGCWACCG | S3 | CGGWAGCAGCGCCWGWWCAGGCGCGAWWAWWWCWGCAAAC | OH |
| 5c | WWCCWGCAWACGWGCGCCACCAGAGGCGGAGAAGCAGAWC | S3 | GAWCWGCWWCWCCGCCWCWGGWGGCGCACGWAWGCAGGAA | OH |
| 6a | AWWACGAAACCACWCCCGCGCWWCGCGGACWWCWGGCAGA | S3 | WCWGCCAGAAGWCCGCGAAGCGCGGGAGWGGWWWCGWAAW | C3 |
| 6b | GWWWGCAGAAAWAAWCGCGCCWGAACAGGCGCWGCWACCG | S3 | CGGWAGCAGCGCCWGWWCAGGCGCGAWWAWWWCWGCAAAC | C3 |
| 6c | WWCCWGCAWACGWGCGCCACCAGAGGCGGAGAAGCAGAWC | S3 | GAWCWGCWWCWCCGCCWCWGGWGGCGCACGWAWGCAGGAA | C3 |
| 7a | AWWACGAAACCACWCCCGCGCWWCGCGGACWWCWGGCAGA | S9 | WCWGCCAGAAGWCCGCGAAGCGCGGGAGWGGWWWCGWAAW | C3 |
| 7b | GWWWGCAGAAAWAAWCGCGCCWGAACAGGCGCWGCWACCG | S9 | CGGWAGCAGCGCCWGWWCAGGCGCGAWWAWWWCWGCAAAC | C3 |
| 7c | WWCCWGCAWACGWGCGCCACCAGAGGCGGAGAAGCAGAWC | S9 | GAWCWGCWWCWCCGCCWCWGGWGGCGCACGWAWGCAGGAA | C3 |
| 8a | ATWACGAAACCACTCCCGCGCWWCGCGGACTTCWGGCAGA | S3 | TCWGCCAGAAGWCCGCGAAGCGCGGGAGTGGTWTCGWAAT | C3 |
| 8b | GTWWGCAGAAATAATCGCGCCWGAACAGGCGCTGCWACCG | S3 | CGGWAGCAGCGCCTGTWCAGGCGCGATTAWWTCTGCAAAC | C3 |
| 8c | TWCCTGCATACGWGCGCCACCAGAGGCGGAGAAGCAGATC | S3 | GAWCTGCTWCTCCGCCTCWGGTGGCGCACGWATGCAGGAA | C3 |

### Selective stabilizing and destabilizing effects of W base

The following oligonucleotides are made using phosphoramidite reagents from Glen Research, in particular, dW phosphoramidite (cat. no 10-1527) and 2-Amino-dA-CE Phosphoramidite (cat. no 10-1085-02). Synthesis and deprotection of modified oligonucleotides are performed by following Glen Research's recommendations for dW phosphoramidite and 2-Amino-dA-CE Phosphoramidites. 2-Amino-dA-CE Phosphoramidite is represented by A' and dW phosphoramidite is represented by T' in the following oligonucleotide sequences.

### Complementary 20-mers

| ODN | Bases | Sequence |
|---|---|---|
| 1a | A and T | 5'-GTAAGAGAATTATGCAGTGC |
| 1b | A' and T' | 5'-GT'A'A'GA'GA'A'T'T'A'T'GCA'GT'GC |
| 1c | A' and T | 5'-GTA'A'GA'GA'A'TTA'TGCA'GTGC |
| 1d | A and T' | 5'-GT'AAGAGAAT'T'AT'GCAGT'GC |
| | | |
| 2a | A and T | 5'-GCACTGCATAATTCTCTTAC |
| 2b | A' and T' | 5'-GCA'CT'GCA'T'A'A'T'T'CT'CT'T'A'C |
| 2c | A' and T | 5'-GCA'CTGCA'TA'A'TTCTCTTA'C |
| 2d | A and T' | 5'-GCACT'GCAT'AAT'T'CT'CT'T'AC |

The melting data ^{a} is collected and summarized in the format shown in Table 3.

**TABLE 3**

| **MELTING TRANSITION TEMPERATURES AND FREE ENERGIES FOR HYBRIDS SUBSTITUTED WITH 2-AMINOADENINE AND/OR W BASE ANALOGS.** | | | | |
|---|---|---|---|---|
| hybrid | A-T doublets*^{b}* | *T*ₘ*^{c}* (°C) | -Δ*G*°₃₇*^{c}* (kcal/mol) | -Δ*G*°₆₀*^{c}* (kcal/mol) |
| 1a-2a | A-T | 55 | 16.2 | 8.1 |
| 1b-2a | A-T', A'-T | | | |
| 1a-2b | A-T', A'-T | | | |
| 1b-2b | A'-T' | | | |

| hybrid | A-T doublets*^{b}* | *T*ₘ*^{c}* (°C) | -Δ*G*°₃₇*^{c}* (kcal/mol) | -Δ*G*°₆₀*^{c}* (kcal/mol) |
|---|---|---|---|---|
| 1a-2c | A-T, A'-T | | | |
| 1a-2d | A-T, A-T' | | | |
| 1b-2c | A'-T, A'-T' | | | |
| 1b-2d | A-T', A'-T' | | | |
| 1c-2a | A-T, A'-T | | | |
| 1c-2b | A'-T, A'-T' | | | |
| 1c-2c | A'-T | | | |
| 1c-2d | A-T, A'-T' | | | |
| 1d-2a | A-T, A-T' | | | |
| 1d-2b | A-T', A'-T' | | | |
| 1d-2c | A-T, A'-T' | | | |
| 1d-2d | A-T' | | | |

| | | | | |
|---|---|---|---|---|
| ^{a} Determined in 200 mM NaCl, 0.1 mM EDTA, and 10 mM Na2HPO4 (pH 7.0) with a 16 µM total nucleotide concentration. ^{b} The presence of A-T, A'-T, A-T', and A'-T' doublets in each hybrid is indicated. ^{c} Each reported value for Tm and free energy is an average of at least three separate experiments; uncertainties in Tm values and in free energies are estimated at ± 1.0 °C and ± 15%, respectively. | | | | |

### Enrichment experiments

Probe sets 1a-c - 8a-c are tested in enrichment experiments. Each experiment is performed in the presence of tree corresponding SBC probes targeting both strands in Seq. 1, Seq. 2 and Seq. 3 duplexes. The results initially are evaluated by PCR.

Selected probe sets that demonstrated best performance are subjected to a sequencing library prep and evaluated by Illumina sequencing in comparison to the libraries obtained without enrichment.

### Strand exchange promoted by RecA enzyme

Hybridization and strand exchange of SBC probes with the targeted dsDNA Seq. 1-3 is performed according to protocols published in S. C. Kowalczykowski and R. A. Krupp, Proc Natl Acad Sci U S A. 1995 Apr 11; 92(8): 3478-3482.

The results initially are evaluated by PCR and compared with the results without recA treatment at strand exchange times 1 hr, 4 hr, 8 hr and 16 hr.

Selected probe sets that demonstrated best performance are subjected to a sequencing library prep and evaluated by Illumina sequencing in comparison to the libraries obtained without enrichment.

## Claims

1. A selectively binding complementary (SBC) oligodeoxynucleotide derivative having the structure:
5'-(Affinity tag)-(SBC sequence 2) - (Spacer) - (SBC sequence 1)-(Blocking group or OH)-3'
wherein
the 3'-blocking group is no blocking (3'-OH), or any alkene diol, or another biotin with a linker, or stabilizing group;
SBC sequences 1 and 2, each is 5 - 100 bases long with strands complementary to a selected region of larger targeted dsDNA, such that the targeted DNA preserves a partial double stranded structure after invasion of SBC probe; and
5'-(Affinity tag) is an affinity tag connected to 5'-end of the probe by a linker.

2. The oligonucleotide derivative of Claim 1, wherein the sequence comprises 2-sT - 2-amA base pairs.

3. The oligonucleotide derivative of Claim 1, wherein the sequence comprises W - 2-amA base pairs.

4. The oligonucleotide derivative of Claim 1, wherein the sequence comprises both 2-sT - 2-amA and W - 2-amA base pairs.

5. The oligonucleotide derivative of Claim 1, wherein the alkene diol is OCH2CH2CH2OH.

6. The oligonucleotide derivative of Claim 1, wherein the stabilizing group is selected from MGB, or intercalators such as phenazinium, acridine, pyrene

7. A method for enriching targeted dsDNA in a DNA sample, comprising:
(a) mixing a selectively binding complementary (SBC) probe with dsDNA extracted from sample or cDNA produced from corresponding RNA, targeted and non-targeted, wherein the SBC probe is composed of two complementary weakly bound strands tethered with a linker and containing an affinity tag;
(b) hybridizing each strand of SBC probe with the targeted region of dsDNA and forming two strong double stand areas (double D-loop) within the targeted dsDNA or cDNA;
(c) treating the mixture with affinity partner-labeled beads (or other solid phase or substrate) and binding the hybridized affinity tagged probe to the beads;
(d) separating the beads containing targeted DNA bound to the beads from non-targeted DNA in the solution and washing the beads; and
(e) melting off the probe while protecting the rest of the targeted DNA in a double stranded form to provide an enriched dsDNA pool.

8. The method of Claim 7, further comprising subj ecting the enriched dsDNA pool to standard library prep for the sequencing.

9. The method of Claims 7 or 8, wherein the SBC probe is an oligodeoxynucleotide derivative of any one of Claims 1-6.

10. The method of any one of Claims 7-9, wherein recA enzyme is used strand exchange of SBC probe with targeted dsDNA for capturing and enrichment.

11. Use of a selectively binding complementary oligodeoxynucleotide derivative of any one of Claims 1-6 for enriching targeted dsDNA in a sample for sequencing-based detection and genomic profiling of the targeted dsDNA.

## Patentansprüche

1. Selektiv bindendes komplementäres (SBC) Oligodesoxynukleotidderivat mit der Struktur:
5'-(Affinitäts-Tag)-(SBC-Sequenz 2) - (Spacer) - (SBC-Sequenz 1)- (Blockierungsgruppe oder OH)-3',
wobei
die 3'-Blockierungsgruppe nicht blockierendes (3'-OH), oder irgendein Alkendiol, oder ein anderes Biotin mit einem Linker, oder Stabilisierungsgruppe ist;
SBC-Sequenzen 1 und 2 jeweils 5-100 Basen lang sind, mit zu einer ausgewählten Region von größerer angezielter dsDNA komplementären Strängen, so dass die angezielte DNA eine partielle doppelsträngige Struktur nach Invasion einer SBC-Sonde präserviert; und
5'-(Affinitäts-Tag) ein Affinitäts-Tag ist, der mit dem 5'-Ende der Sonde durch einen Linker verbunden ist.

2. Oligonukleotidderivat nach Anspruch 1, wobei die Sequenz 2-sT - 2-amA-Basenpaare umfasst.

3. Oligonukleotidderivat nach Anspruch 1, wobei die Sequenz W - 2-amA-Basenpaare umfasst.

4. Oligonukleotidderivat nach Anspruch 1, wobei die Sequenz sowohl 2-sT - 2-amA als auch W - 2-amA-Basenpaare umfasst.

5. Oligonukleotidderivat nach Anspruch 1, wobei das Alkendiol OCH2CH2CH2OH ist.

6. Oligonukleotidderivat nach Anspruch 1, wobei die Stabilisierungsgruppe aus MGB, oder Interkalatoren wie z. B. Phenazinium, Acridin, Pyren, ausgewählt ist.

7. Verfahren zur Anreicherung angezielter dsDNA in einer DNA-Probe, umfassend:
(a) Mischen einer selektiv bindenden komplementären (SBC) Sonde mit aus einer Probe extrahierter dsDNA oder aus entsprechender RNA produzierter cDNA, angezielt und nicht angezielt, wobei die SBC-Sonde aus zwei komplementären schwach gebundenen Strängen, verknüpft mit einem Linker und enthaltend ein Affinitäts-Tag, besteht;
(b) Hybridisieren jedes Strangs der SBC-Sonde mit der angezielten Region der dsDNA und Bilden zweier starker Doppelstrangbereiche (Doppel-D-Loop) innerhalb der angezielten dsDNA oder cDNA;
(c) Behandeln der Mischung mit Affinitätspartner-markierten Beads (oder anderer fester Phase oder anderem festem Substrat) und Binden der hybridisierten Affinitäts-getaggten Sonde an die Beads;
(d) Trennen der Beads, enthaltend angezielte DNA, gebunden an die Beads, von nicht angezielter DNA in der Lösung und Waschen der Beads; und
(e) Wegschmelzen der Sonde, während der Rest der angezielten DNA in einer doppelsträngigen Form geschützt wird, um einen angereicherten dsDNA-Pool bereitzustellen.

8. Verfahren nach Anspruch 7, ferner umfassend Unterziehen des angereicherten dsDNA-Pools einer Standardbibliothekpräparation für die Sequenzierung.

9. Verfahren nach Ansprüchen 7 oder 8, wobei die SBC-Sonde ein Oligodesoxynukleotidderivat nach einem der Ansprüche 1 bis 6 ist.

10. Verfahren nach einem der Ansprüche 7 bis 9, wobei recA-Enzym für Strangaustausch der SBC-Sonde mit angezielter dsDNA zwecks Einfangens und Anreicherung verwendet wird.

11. Verwendung eines selektiv bindenden komplementären Oligodesoxynukleotidderivats nach einem der Ansprüche 1 bis 6 zur Anreicherung angezielter dsDNA in einer Probe für Sequenzierungs-basierten Nachweis und genomische Profilierung der angezielten dsDNA.

## Revendications

1. Dérivé d'oligodésoxynucléotide complémentaire à liaison sélective (SBC) présentant la structure :
5'-(tag d'affinité)-(séquence SBC 2) - (Bras espaceur) - (Séquence SBC 1)- (Groupe de blocage ou OH)-3'
dans lequel
le groupe de blocage-3' est non bloquant (3'-OH), ou un quelconque diol alcène, ou une autre biotine avec un bras de liaison, ou un groupe de stabilisation ;
des séquences SBC 1 et 2, chacune de 5-100 bases avec des brins complémentaires à une région sélectionnée d'ADNdb ciblé supérieur, de sorte que l'ADN ciblé préserve une structure double brin partielle après invasion d'une sonde SBC ; et
le 5'-(tag d'affinité) est un tag d'affinité relié à une extrémité 5' de la sonde par un bras de liaison.

2. Dérivé d'oligonucléotide selon la revendication 1, dans lequel la séquence comprend des paires de bases 2-sT - 2amA.

3. Dérivé d'oligonucléotide selon la revendication 1, dans lequel la séquence comprend des paires de bases W - 2amA.

4. Dérivé d'oligonucléotide selon la revendication 1, dans lequel la séquence comprend à la fois des paires de bases 2-sT - 2amA et W - 2amA.

5. Dérivé d'oligonucléotide selon la revendication 1, dans lequel l'alcène diol est un OCH2CH2CH2OH.

6. Dérivé d'oligonucléotide selon la revendication 1, dans lequel le groupe de stabilisation est sélectionné parmi un MGB, ou des agents d'intercalation comme le phénazinium, l'acridine, le pyrène.

7. Procédé permettant d'enrichir l'ADNdb ciblé dans un échantillon d'ADN, comprenant :
(a) le fait de mélanger une sonde complémentaire à liaison sélective (SBC) avec de l'ADNdb extrait d'un échantillon ou d'ADNc produit à partir d'ARN correspondant, ciblé et non-ciblé, dans lequel la sonde SBC est composée de deux brins faiblement liés complémentaires attachés par un bras de liaison et contenant un tag d'affinité ;
(b) l'hybridation de chaque brin de sonde SBC avec la région ciblée d'ADNdb et la formation de deux zones doubles brins fortes (double boucle D) dans l'ADNdb ou l'ADNc ciblé ;
(c) le traitement du mélange avec des perles marquées par un partenaire d'affinité (ou une autre phase solide ou substrat) et la liaison de la sonde marquée par affinité hybridée aux perles ;
(d) la séparation des perles contenant l'ADN ciblé lié aux perles de l'ADN non-ciblé dans la solution et le lavage des perles ; et
(e) le fait de faire fondre la sonde pour l'éliminer tout en protégeant le reste de l'ADN ciblé sous une forme à double brin pour fournir un pool d'ADNdb enrichi.

8. Procédé selon la revendication 7, comprenant en outre la soumission du pool d'ADNdb enrichi à une préparation de banque standard pour le séquençage.

9. Procédé selon les revendications 7 ou 8, dans lequel la sonde SBC est un dérivé d'oligodésoxynucléotide selon l'une quelconque des revendications 1 à 6.

10. Procédé selon l'une quelconque des revendications 7 à 9, dans lequel l'enzyme recA est utilisée par échange de brin de la sonde SBC avec l'ADNdb ciblé pour capture et enrichissement.

11. Utilisation d'un dérivé d'oligodésoxynucléotide complémentaire à liaison sélective selon l'une quelconque des revendications 1 à 6 pour enrichir l'ADNdb ciblé dans un échantillon pour une détection basée sur le séquençage et le profilage génomique de l'ADNdb ciblé.
